# EUROPEAN PATENT APPLICATION

(11) **EP 4 035 676 A1**
(43) Date of publication of application: **03.08.2022**
(21) Application number: 21386011.7
(22) Date of filing: 01.02.2021
(51) Int. Cl.: A61K 39/12, A61K 39/215, A61K 39/39, A61P 31/14, A61K 39/00

(54) **VACCINE COMPOSITIONS**

(71) Applicant: Oxford University Innovation Limited, Oxford, OX2 0JB (GB)
(72) Inventor: The designation of the inventor has not yet been filed
(74) Representative: J A Kemp LLP

(57) **Abstract**

The invention describes vaccine compositions containing particles having a polypeptide shell and a water-immiscible core. The polypeptide shell may comprise one or more pathogenic antigen proteins and/or one or more adjuvant polypeptides. Administration of the composition generates an immune response to the polypeptide contained in the shell. Adjuvant may be comprised in the water-immiscible core of the particle. The particles are therefore useful in methods of vaccination.

## Description

### FIELD OF THE INVENTION

The present invention relates to a vaccine composition, comprising polypeptide particles formed of a water-immiscible core and a polypeptide shell. The compositions can be used in methods of vaccination.

### BACKGROUND AND SUMMARY OF INVENTION

The present inventors have found that polypeptide particles having a water-immiscible liquid core and a polypeptide shell can generate a significant immune response to the polypeptide present in the shell. The particles can be produced with pathogenic antigen protein comprised in the polypeptide shell and thereby act as vaccine particles. The particles can alternatively contain an adjuvant polypeptide in the polypeptide shell. Such particles, when co-administered with a vaccine, can enhance the immune response to the vaccine particles.

Protein nanoparticles have found use in recent years as drug delivery systems. For instance, cancer therapeutics such as paclitaxel may be delivered in the form of a protein nanoparticle having an oil core. Such particles are commercially available as Abraxane^{®}. In these types of particle, the protein is used as a delivery vehicle to deliver the chosen drug to the desired site. Immune response to the protein delivery vehicle would cause an adverse reaction in the patient and accordingly such drug delivery vehicles are produced in such a way as to minimise any immune response, by use of non-immunomodulatory proteins such as human serum albumin.

The present inventors, however, have surprisingly found that core-shell particles having a water-immiscible core and a protein shell generate an immune response to the protein in the shell which is greater than that of the corresponding native protein. Whilst such immune response must be minimised in the context of drug delivery, it can be used to advantage in immunisation. Thus, the present inventors provide vaccine compositions comprising such protein particles, wherein the protein shell comprises a pathogenic antigen protein or an adjuvant polypeptide. These particles generate an immunologic response and are therefore useful in vaccines compositions.

The present invention therefore provides, in a first aspect (1):
1. A vaccine composition comprising a plurality of polypeptide particles and one or more pharmaceutically acceptable carriers or diluents, wherein the particles comprise:
   - a water immiscible liquid core optionally comprising one or more adjuvants; and
   - a polypeptide shell.
The invention also provides the following aspects:
2. A vaccine composition according to aspect 1, wherein the polypeptide comprises a pathogenic antigen protein and/or wherein the composition further comprises a vaccine, preferably a DNA or RNA vaccine.
3. A vaccine composition according to aspect 1 or aspect 2, wherein any indentations on the surface of the polypeptide particles have a maximum dimension of 50nm.
4. A vaccine composition according to any one of aspects 1 to 3, wherein the particles are substantially spherical.
5. A vaccine composition according to any one of the preceding aspects, wherein the polypeptide particles have a mean particle size in the range of from 100nm to 1000nm, preferably from 200nm to 700nm.
6. A vaccine composition according to any one of the preceding aspects, wherein each polypeptide is conjugated to one or more ligands selected from a peptide, a protein, a sugar, a nanoparticle and a nucleic acid.
7. A vaccine composition according to any one of the preceding aspects, wherein the water-immiscible core comprises one or more adjuvants.
8. A vaccine composition according to aspect 7, wherein the one or more adjuvants are selected from imiquimod and squalene.
9. A vaccine composition according to any one of the preceding aspects, wherein the polypeptide comprises a pathogenic antigen protein, preferably wherein the pathogenic antigen protein is selected from a covid spike protein, HepB surface antigen protein, hemagglutinin, an influenza neuraminidase, filamentous hemagglutinin, pneumococcal surface protein A, Neisserial adhesin A, Neisserial Heparin Binding Antigen, factor H binding protein, and HPV-16 E6 or E7 fusion protein.
10. A vaccine composition according to any one of the preceding aspects, wherein the polypeptide comprises an adjuvant polypeptide, preferably wherein the adjuvant polypeptide is one or more polypeptides selected from non-human albumin proteins, sFLT ligand, cytokines and chemokines, macrophage colony stimulating factor (M-CSF), tumour necrosis factor (TNF), and granulocyte and macrophage colony stimulating factor (GM-CSF)
11. A vaccine composition according to any one of the preceding aspects, wherein the composition further comprises a DNA or RNA vaccine.
12. A polypeptide particle comprising:
   - a water immiscible liquid core optionally comprising one or more adjuvants, the adjuvants being as defined in aspect 7 or aspect 8; and
   - a polypeptide shell comprising a pathogenic antigen protein as defined in aspect 9;
   preferably wherein any indentations on the surface of the polypeptide particle have a maximum dimension of 50nm.
13. A polypeptide particle comprising:
   - a water immiscible liquid core optionally comprising one or more adjuvants, the adjuvants being as defined in aspect 7 or aspect 8; and
   - a polypeptide shell comprising an adjuvant polypeptide as defined in aspect 10;
   preferably wherein any indentations on the surface of the polypeptide particle have a maximum dimension of 50nm.
14. A pharmaceutical adjuvant composition comprising a plurality of particles according to aspect 13, together with one or more pharmaceutically acceptable carriers or diluents.
15. A polypeptide particle or composition according to any one of aspects 12 to 14, which particle is as defined in any one of aspects 4 to 6.
16. A method of producing a polypeptide particle according to any one of aspects 12, 13 or 15, comprising:
   - providing a water-immiscible phase optionally comprising one or more adjuvants as defined in aspect 7 or aspect 8;
   - mixing said water-immiscible phase with an aqueous solution of at least one polypeptide, wherein the at least one polypeptide comprises a pathogenic antigen protein as defined in aspect 9 and/or an adjuvant polypeptide as defined in aspect 10; and
   - cross-linking the polypeptide to generate a particle having a core comprising the water-immiscible phase and a shell comprising the at least one polypeptide.
17. A method according to aspect 16, which further comprises a step of purification of the particles.
18. A polypeptide particle or composition according to any one of aspects 1 to 15, for use in a method of vaccination.
19. A polypeptide particle or composition for use according to aspect 18, wherein the polypeptide particle or composition is administered by injection, by oral administration or by intra-nasal administration.
20. A polypeptide particle or composition for use according to aspect 19, wherein the polypeptide particle or composition is administered by intravenous, intradermal, intramuscular or subcutaneous injection.
21. A polypeptide particle or composition for use according to any one of aspects 18 to 20, wherein the polypeptide particle or composition is administered together with a DNA or RNA vaccine.
22. Use of a polypeptide particle or composition according to any one of aspects 1 to 15 in the manufacture of a medicament for use in a method of vaccination, in particular wherein the method is as set out in any one of aspects 19 or 20.
23. Use according to aspect 22, wherein the polypeptide particle or composition is administered together with a DNA or RNA vaccine.
24. A method of vaccination of a subject, which method comprises administering to said subject a prophylactically effective amount of a polypeptide particle or composition according to any one of aspects 1 to 15, in particular wherein the method is as set out in any one of aspects 19 or 20.
25. A method according to aspect 24, wherein the polypeptide particle or composition is administered together with a DNA or RNA vaccine.

### BRIEF DESCRIPTION OF THE FIGURES

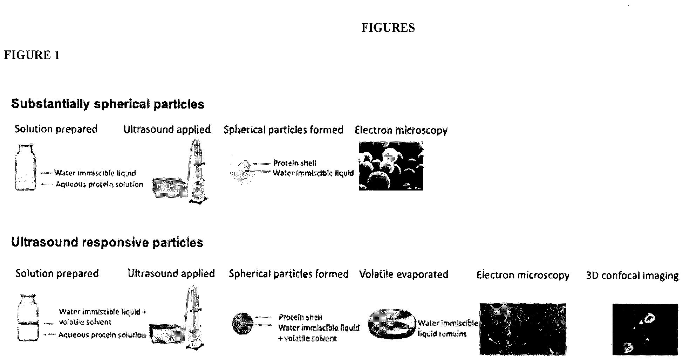

Figure 1 provides a schematic representation of the process used to make the particles of the invention.

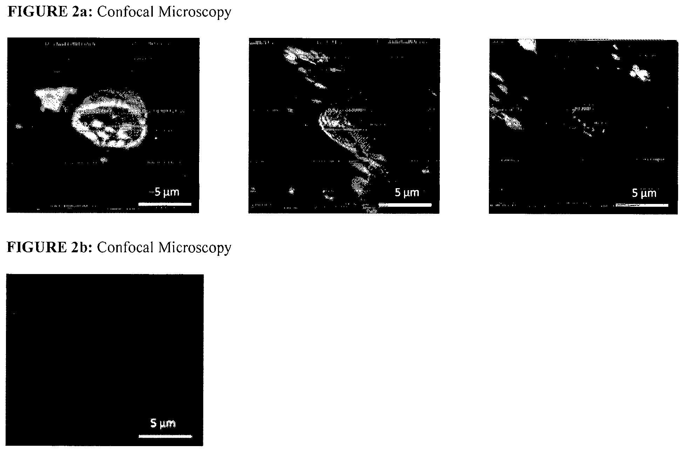

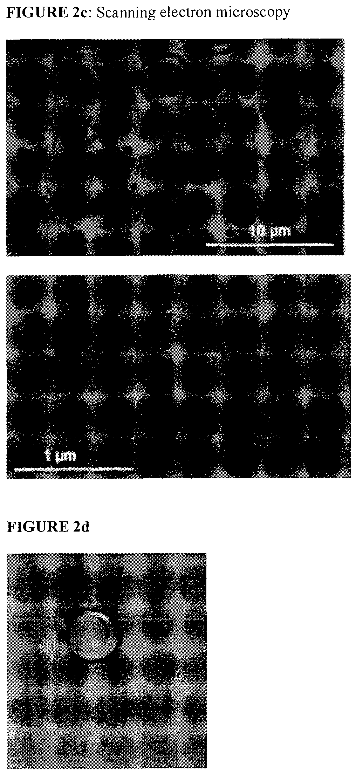

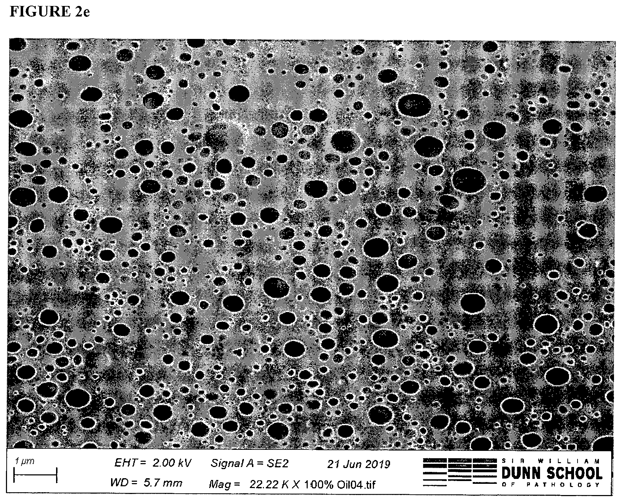

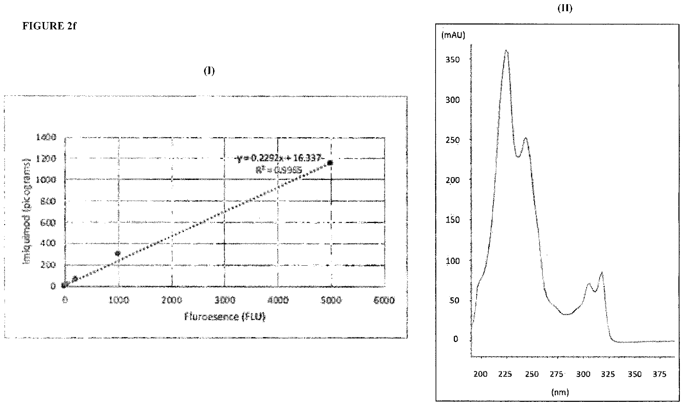

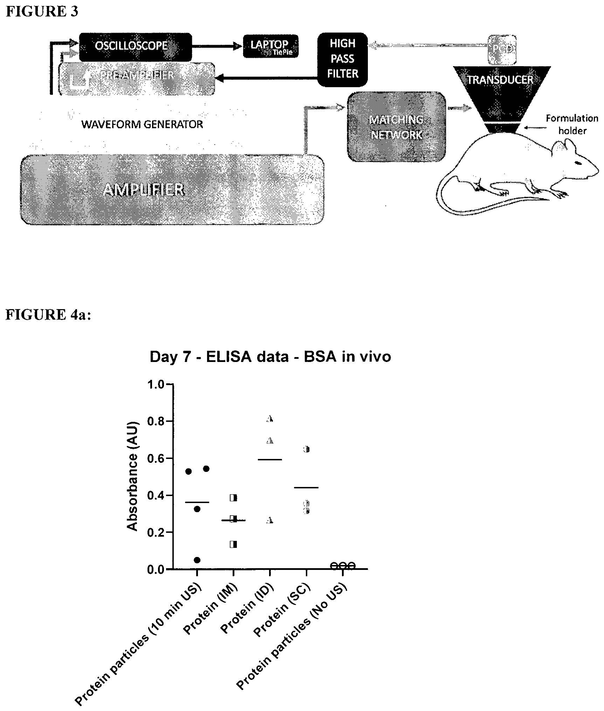

Figures 2a and b show 3D reconstruction of Z-stacks generated via spinning disc confocal microscopy with particles pre-conjugated with AlexaFluor 488 NHS (Fig 2a) and CY5 NHS (Fig 3b); Figure 2c shows scanning electron microscopy (SEM) images of particles according to the invention; Figure 2d shows a light microscopy image of a particle according to the invention; Figure 2e shows an SEM image of substantially spherical particles according to the invention; Figure 2f(I) shows the quantity of imiquimod plotted against corresponding fluorescence peak area detected (Ex260nm Em340nm); Figure 2f(II) shows the UV absorbance profile of imiquimod.
Figure 3 provides a schematic of the ultrasound hardware used in in vivo cavitation experiments
Figure 4 shows: (a) Day-7 ELISA comparing the immune response to BSA protein, following delivery of particles made from BSA both transdermally and via a range of injections; (b) Avidity ELISA to quantify the binding affinity of antibodies generated.
Figure 5 shows results of an ELISA quantifying the binding of native Cetuximab protein and protein particles generated from Cetuximab to EGFR (native receptor).
Figure 6 shows the results of an ELISA specific to covid spike protein taken 21 days after administration of covid spike protein via various modes of administration.

### DETAILED DESCRIPTION OF THE INVENTION

### Definitions

Particle size as defined herein is the maximum distance across a particle, i.e. the diameter in the case of a spherical particle. Although the particles defined herein are not necessarily spherical in shape, the particle size may be referred to herein as a particle diameter and both terms have the same meaning.

Particle size in the case of a composition containing a plurality of particles according to the invention is defined as the mean particle size. Mean particle size within a composition may be measured by a variety of techniques known to the skilled person (Malvern Nanosight, Beckman Coulter particle size, dynamic light scattering (DLS)). Typically the mean particle size is defined as the hydrodynamic diameter, as measured by dynamic light scattering (DLS). Particle size of an individual particle may be measured by scanning electron microscopy (SEM).

The shell thickness is the average thickness of the polypeptide shell and can be determined by SEM or TEM.

As used herein, the term "polypeptide" refers to a full-length protein, a portion of a protein, or a peptide characterized as a string of amino acids. Typically a polypeptide comprises at least 25, or 30, or 35, or 40, or 45, or 50 or 55 or 60 amino acids. For example, a polypeptide may contain 100 or more amino acids. As used herein, the term "protein" refers to a full length protein or a fragment of a protein.

The terms "fragment", "fragment of a protein" or "fragment of a polypeptide" as used herein refer to a string of amino acids or an amino acid sequence typically of reduced length relative to the or a reference protein or polypeptide and comprising, over the common portion, an amino acid sequence identical to the reference protein or polypeptide. In some cases the fragments referred to herein may be between 8 or 9 and 20, or 25, or 30, or 35, or 40, or 45, or 50 amino acids.

The term volatile as used herein indicates that a substance has a vapour pressure at 25°C greater than that of water (which has a vapour pressure about 3kPa) at the same temperature. Typically, a volatile substance, or a volatile component, is capable of vaporising at room temperature (25°C). A volatile component as used herein is thus a liquid, typically an organic solvent, which has a vapour pressure of at least 3 kPa, preferably at least 3.5 kPa, for example at least 5 kPa, at least 10 kPa or at least 15 kPa at 25°C.

Non-volatile indicates that a substance does not or substantially does not vaporise at room temperature (25°C). Non-volatile components are thus typically liquids, e.g. oils, which have a vapour pressure at 25°C which is no more than that of water (which has a vapour pressure about 3kPa) at the same temperature. A non-volatile component as used herein typically has a vapour pressure at 25°C of less than 3 kPa, preferably less than 2.5kPa, e.g. less than 2 kPa.

As described herein, the particles are useful in methods of vaccination of a subject. The subject is a human or animal subject. In one aspect, the subject to be treated is a mammal, in particular a human. However, it may be non-human. Preferred non-human animals include, but are not limited to, primates, such as marmosets or monkeys, commercially farmed animals, such as horses, cows, sheep or pigs, and pets, such as dogs, cats, mice, rats, guinea pigs, ferrets, gerbils or hamsters.

The term "vaccine composition" as used herein refers to a composition which can be used for the vaccination of a subject. A vaccine composition contains a vaccine, i.e. an active agent suitable for inducing an immune response in a subject. A vaccine may, for example, be a DNA or RNA vaccine or an antigen for inducing an immune response to a pathogen. For example, a vaccine composition may comprise a DNA or RNA vaccine and/or a pathogenic antigen protein. The vaccine may be a part of the polypeptide shell of the particle itself, a ligand attached to the polypeptide shell of the particle, and/or may be provided as a separate component in the composition. Vaccine compositions may additionally comprise an adjuvant.

The term "vaccination" as used herein refers to a prophylactic treatment. Administration is typically in a "prophylactically effective amount", this being sufficient to result in a clinical response or to show clinical benefit to the individual, e.g. an effective amount to prevent or delay onset of a disease or condition.

The term "adjuvant" as used herein refers to a substance which is used to enhance immunomodulation. Adjuvants include both small molecule and protein-based substances which act to boost the immune response, e.g. to a vaccine. Immuno-stimulators such as cytokines and chemokines are examples of adjuvants as used herein. The term "adjuvant polypeptide" as used herein is a polypeptide capable of acting as an adjuvant. The term "adjuvant composition" as used herein is a composition comprising an adjuvant and which may optionally also contain a vaccine.

### Polypeptide particle morphology

The particles described herein typically have an average size of 8000nm or less, e.g. 5000nm or less, preferably they are nanoparticles having a particle size of 1000nm or less. Preferably, the particles have a size of from 100nm to 8000nm, e.g. from 100nm to 5000nm, preferably from 100nm to 1000nm. For example, the particles may have a size of from 200nm to 700nm, for example around 500nm. In one aspect, the particles have a size which is 450nm or less. Preferably, the mean particle size of a composition comprising one or more particles of the invention is from 100nm to 8000nm, e.g. from 100nm to 5000nm, preferably from 100nm to 1000nm, more preferably from 200nm to 700nm, for example around 500nm. In one aspect, the composition contains particles having a mean particle size which is 450nm or less. Particle size can be controlled, for example, by size filtration of particles.

Particles having indentations in their surface sufficient to trap a gas bubble may be ultrasound-responsive particles. Such particles are capable of generating a cavitation response, typically an inertial cavitation response, when exposed to ultrasound in the presence of a fluid (e.g. the liquid of an injectable vaccine composition). When such particles are exposed to ultrasound, a gas bubble trapped in the indentation may change size or shape, for example it may grow following exposure to the acoustic pressure, and subsequently collapse. This may cause a shock-wave or micro-streaming in the surrounding fluid. Typically, the particles of the present invention do not generate inertial cavitation on response to ultrasound, i.e. they are typically not ultrasound-responsive particles.

Inertial cavitation may be assessed as the presence of broadband noise at a level clearly discernible from the background (e.g. at least 3 times the background root-mean-square noise) after excluding any tonal components. Particles are considered to generate an inertial cavitation response if broadband noise (e.g. measured as described above) is present on application of ultrasound (e.g. at 1.2MPa and 265kHz) to a suspension of particles in water. Typically, the particles of the invention do not generate an inertial cavitation response (i.e. broadband emission) when suspended in water and subject to ultrasound at IMPa and 500kHz. For instance, the particles of the invention do not generate an inertial cavitation response (i.e. broadband emission) when suspended in water and subject to ultrasound at 1.5MPa and 500kHz.

Typically the particles described herein do not have any major indentations in the particle surface, thus they do not have indentations capable of trapping a gas bubble. Thus, the invention typically relates to particles wherein any surface indentations have an opening size (the maximum dimension across the opening at the surface of the particle) of less than 50nm, preferably less than 20nm, more preferably less than 10nm. Typically, any indentations on such particles have a depth of less than 10nm. Typically, any indentations in the particles have a depth of less than 10% of particle size. Typically, such particles do not have any surface indentations.

Typically these particles are substantially spherical. Substantially spherical as used herein is a term used to refer to particles which are not capable of generating inertial cavitation on response to ultrasound. Thus, substantially spherical particles can have a varying 3D shape, which does not need to be exactly spherical and may, for example, be a spheroid or ellipsoid. Such particles generally do not have the ability to entrap a gas bubble within their structure, e.g. within any indentations in the particle. Such particles may have minor surface features but generally do not have indentations capable of trapping a gas bubble, e.g. indentations having a depth of 10nm or more and/or an opening size of 50nm or more.

The particles of the invention have a core comprising a water-immiscible phase comprising one or a mixture of water-immiscible liquids (hereinafter a "water-immiscible core"). The content of water-immiscible core as a proportion of the particle may vary widely. For example, the particle may comprise from 0.1 to 70% by weight water-immiscible core and from 30 to 99.9% by weight polypeptide shell.

The water-immiscible liquid is typically a non-volatile, water-immiscible liquid (also referred to herein as a non-volatile component). Typically, the non-volatile component is an oil. The nature of the non-volatile component is not particularly limited and any biocompatible, pharmaceutically acceptable oil or mixture thereof can be used. Suitable components include sunflower oil, olive oil, soybean oil, coconut oil, safflower oil, cotton seed oil, sesame oil, orange oil, limonene oil, polyethylene glycols, or other non-volatile organic solvents and combinations of two or more of these oils. Sunflower oil, olive oil or a combination of these oils is preferred. The biocompatible, pharmaceutically acceptable oil is typically not itself pharmaceutically active.

In one aspect, the water-immiscible core consists essentially of, or consists of, one or more non-volatile components. For example, in one embodiment, the water-immiscible core consists essentially of, or consists of, one or a mixture of pharmaceutically acceptable excipients which are biocompatible, pharmaceutically acceptable oils, typically the biocompatible, pharmaceutically acceptable oils listed above. In a preferred aspect of this embodiment, the water-immiscible core consists essentially of, preferably consists of sunflower oil, olive oil or a combination of these oils. In this context, the expression "consists essentially of' means that the water-immiscible core typically contains no more than 5% by weight (e.g. no more than 2%, or no more than 1% by weight, preferably no more than 0.5% by weight, more preferably no more than 0.1% or 0.01% by weight) of other substances, e.g. substances other than non-volatile components, with reference to the total weight of the core. Thus, the water-immiscible core may comprise at least 95% by weight, preferably at least 98%, 99% by weight, more preferably at least 99.5 or 99.9% and most preferably at least 99.99% by weight of one or more pharmaceutically acceptable excipients which are biocompatible, pharmaceutically acceptable oils. The biocompatible, pharmaceutically acceptable oils are typically non-pharmaceutically active, and are preferably selected from those oils set out above.

In one embodiment, the core of the particle comprises one or more adjuvants. The one or more adjuvants may, for example, be one or more selected from adjuvants soluble in an oilbased solvent. Suitable examples include aluminum salts such as alum, aluminum hydroxide or aluminum phosphate, calcium salts (e.g. calcium phosphate hydroxide), iron salts, zinc salts, an insoluble suspension of acylated tyrosine, acylated sugars, cationically or anionically derivatised saccharides, polyphosphazenes, biodegradable microspheres, monophosphoryl lipid A (MPL), lipopolysaccharides, lipid A derivatives (e.g. of reduced toxicity), 3-O-deacylated MPL [3D-MPL], detergents, e.g. quil A, Saponin, QS21, Freund's Complete Adjuvant, Freund's Incomplete Adjuvant (Difco Laboratories, Detroit, Mich.), Merck Adjuvant 65 (Merck and Company, Inc., Rahway, N.J.), AS-2 (Smith-Kline Beecham, Philadelphia, Pa.), CpG oligonucleotides, oils, e.g. squalene, mineral oils e.g. paraffin oil, food-based oils e.g. adjuvant 65 (derived from peanut oil), bacterial products, e.g. killed bacteria selected from *Bordatellapertussis, Mycobacterium bovis,* toxoids, bioadhesives and mucoadhesives, microparticles, liposomes, polyoxyethylene ether formulations, polyoxyethylene ester formulations, muramyl peptides or imidazoquinolone compounds (e.g. imiquamod and its homologues). Human immunomodulators suitable for use as adjuvants include cytokines such as interleukins (e.g. IL-1, IL-2, IL-4, IL-5, IL-6, IL-7, IL-12, etc), macrophage colony stimulating factor (M-CSF), tumour necrosis factor (TNF), granulocyte and macrophage colony stimulating factor (GM-CSF). These immunomodulators are further examples of adjuvants which may be incorporated in the core. In a particular embodiment, the core comprises an imidazoquinolone compound, e.g. imiquimod. In one embodiment, the adjuvant is imiquimod or squalene or a mixture thereof.

The particle has a polypeptide shell, which typically surrounds the core. Typically, the polypeptide shell comprises a polypeptide having two or more cysteine residues. This enables the polypeptide to form disulphide cross-linking bonds in order to generate the polypeptide shell. Preferably, the polypeptide contains at least 4 cysteine residues, for example at least 10 cysteine residues. A greater number of cysteine residues, in particular cysteine residues which are accessible on the surface of the polypeptide, generates a greater degree of cross-linking in the polypeptide shell, which may in turn provide greater stability to the structure. Cysteine residues are naturally occurring in many proteins. However, cysteine residues may be introduced into the polypeptide chain, for example by genetic engineering techniques.

The polypeptide shell typically comprises a polypeptide having a molecular weight of at least 5kD, preferably at least 10kD. A wide range of polypeptides having varying molecular weights can be used to form the polypeptide shell. Typically, therefore, the molecular weight of the polypeptide is from 5 to 80kD, preferably from 10 to 50kD.

The polypeptide shell may comprise at least one pathogenic antigen protein. The pathogenic antigen protein may be a full length protein or a fragment thereof. Examples of suitable antigen proteins include fragments of viral proteins or of parasitic proteins. The antigen may relate to, for example, a pathogen selected from Cal09 (flu), a coronavirus (e.g. Beta-coronaviridae or SARS-CoV-2), hepatitis B, *Bordatella pertussis* (whooping cough), *Streptococcus pneumoniae,* a meningococcus bacterium, human papilloma virus (HPV), or *Plasmodium* sporozoites (malaria parasite).

In one preferred aspect, the antigen protein is a viral spike protein, preferably a spike protein of the SARS-CoV-2 protein (e.g. SARS-CoV-2, S1 subunit protein (RBD)). In another aspect, the antigen is a circumsporozoite protein (CSP), a secreted surface protein of the sporozoite parasite. In a further aspect, the antigen is HepB surface antigen protein (HBsAg). In a further aspect, the antigen is associated with the influenza virus and is selected from haemagglutinin and/or an influenza neuraminidase. In another aspect, the antigen is filamentous haemagglutinin (pertussis). In another aspect, the antigen is pneumococcal surface protein A (PspA). In another aspect, the antigen is selected from Neisserial adhesin A (NadA), Neisserial Heparin Binding Antigen (NHBA) and/or factor H binding protein (fHbp). In another aspect, the antigen is an HPV-16 E6/E7 fusion protein. Fragments or genetically modified versions of any of these proteins may also be used.

The polypeptide shell of the particle may comprise two or more different antigen proteins. For example, two or more different antigen proteins of the same pathogen, or two or more antigens of different pathogens. Alternatively or additionally, a composition according to the invention may comprise two or more different particles, wherein the particles comprise different antigen proteins in their polypeptide shell. In this case, the composition may comprise particles having two or more different antigen proteins of the same pathogen, and/or two or more antigens of different pathogens.

In one embodiment, the polypeptide shell comprises an adjuvant polypeptide, such that the polypeptide shell may act as an adjuvant, increasing the immunomodulatory effect of the vaccine. Examples of suitable adjuvant polypeptides include non-human albumin proteins (e.g. bovine serum albumin, mouse serum albumin, ovalbumin), sFLT ligand, cytokines and chemokines such as interleukins (e.g. IL-1, IL-2, 1L-4, IL-5, IL-6, IL-7, IL-12, etc), macrophage colony stimulating factor (M-CSF), tumour necrosis factor (TNF), granulocyte and macrophage colony stimulating factor (GM-CSF). Ovalbumin is a preferred adjuvant protein. A combination of two or more such proteins may be used.

The polypeptide shell may comprise a pathogenic antigen protein in combination with an adjuvant polypeptide. Alternatively, the polypeptide shell may comprise either a pathogenic antigen protein or an adjuvant polypeptide. Where the polypeptide shell comprises only an adjuvant polypeptide, the vaccine composition described herein also comprises a vaccine, e.g. a separate pathogenic antigen protein, a pathogenic antigen protein ligand, or a DNA or RNA vaccine.

Preferably, the polypeptide shell comprises a single polypeptide (i.e. the polypeptide shell contains a single type of polypeptide, including fragments thereof, and does not contain a combination of two or more different polypeptides).

The polypeptide shell is optionally conjugated to one or more ligands. For example, the polypeptide shell may be conjugated to one or more ligands selected from a peptide, a protein, a sugar, a nanoparticle and a nucleic acid. Labelling moieties may also be conjugated to the polypeptide shell. Conjugation to proteins is commonly used for a variety of reasons. For example, the properties of the protein may be modified by conjugation to glycans (sugars) or to further proteins (e.g. antigens) having specific desired properties. In one embodiment, the polypeptide shell is conjugated to one or more adjuvants. Suitable adjuvants include an aluminum salt such as alum, aluminum hydroxide or aluminum phosphate, but may also be a salt of calcium (e.g. calcium phosphate hydroxide), iron or zinc, or may be an insoluble suspension of acylated tyrosine, or acylated sugars, or may be cationically or anionically derivatised saccharides, polyphosphazenes, biodegradable microspheres, monophosphoryl lipid A (MPL), lipopolysaccharides, lipid A derivatives (e.g. of reduced toxicity), 3-O-deacylated MPL [3D-MPL], detergents, e.g. quil A, Saponin, QS21, Freund's Complete Adjuvant, Freund's Incomplete Adjuvant (Difco Laboratories, Detroit, Mich.), Merck Adjuvant 65 (Merck and Company, Inc., Rahway, N.J.), AS-2 (Smith-Kline Beecham, Philadelphia, Pa.), CpG oligonucleotides, oils, e.g. squalene, mineral oils e.g. paraffin oil, food-based oils e.g. adjuvant 65 (derived from peanut oil), bacterial products, e.g. killed bacteria selected from *Bordatella pertussis, Mycobacterium bovis,* toxoids, bioadhesives and mucoadhesives, microparticles, liposomes, polyoxyethylene ether formulations, polyoxyethylene ester formulations, muramyl peptides or imidazoquinolone compounds (e.g. imiquamod and its homologues). Human immunomodulators including cytokines such as interleukins (e.g. IL-1, IL-2, IL-4, IL-5, IL-6, IL-7, IL-12, etc), macrophage colony stimulating factor (M-CSF), tumour necrosis factor (TNF), granulocyte and macrophage colony stimulating factor (GM-CSF) may also be used as adjuvants. Such adjuvants may be modified to include a suitable binding site to enable conjugation to the polypeptide shell.

A common technique for conjugation to proteins, which can be employed in the present invention, is to target lysine residues on the protein surface. Thus, ligands having a group reactive with amine (e.g. NHS) may be conjugated to lysine on the polypeptide shell. However, alternative conjugation chemistries may be employed.

The ligands may be conjugated to the polypeptide shell after particle formation. Alternatively, the ligand may be conjugated to the polypeptide before formation of the particle.

The shell thickness of the polypeptide particle is typically at least 10nm, preferably at least 20nm, for example about 30-40nm. For example, the polypeptide shell thickness may be from 10 to 100nm, preferably from 20 to 60nm. The thickness of the polypeptide nanoparticle shell can be determined by cryo-slicing and TEM imaging.

The particles of the invention are highly stable and can be stored as a suspension in aqueous solution or in solid form. For instance, the particles can be stored for at least a period of several months at 4°C.

### Synthesis

The invention also provides a method of producing a polypeptide particle as described herein, comprising:
- providing a water-immiscible phase optionally comprising one or more adjuvants;
- mixing said water-immiscible phase with an aqueous solution of at least one polypeptide, wherein the at least one polypeptide comprises a pathogenic antigen protein and/or an adjuvant polypeptide; and
- cross-linking the polypeptide to generate a particle having a core comprising the water-immiscible phase and a shell comprising the at least one polypeptide.

The particles are typically produced by first creating a biphasic composition comprising (1) a water-immiscible phase comprising one or more water-immiscible liquids (which are typically one or more non-volatile components) and optionally one or more adjuvants; and (2) an aqueous phase comprising the polypeptide or mixture of polypeptides which is to be present in the polypeptide shell. The aqueous solution typically comprises at least about 0.05% w/v of polypeptide, for example at least about 0.5%, preferably at least about 1% w/v polypeptide. Typically the polypeptide concentration is no higher than about 25% w/v, e.g. no higher than about 15% w/v, preferably no higher than about 10 %w/v. Thus, suitable concentrations of polypeptide in the aqueous phase are from 0.5 to 15% w/v, preferably from 1 to 10% w/v, more preferably about 5% w/v.

The volumetric ratio of water-immiscible phase to aqueous phase in the biphasic mixture is typically about 1:5 to 3:1, for example from 1:4 to 1:1, e.g. about 1:3.

The water-immiscible liquid may initially be used as a solvent for any adjuvant present, such that a solution of adjuvant in the water-immiscible liquid is used to produce the particles of the invention. Any pharmaceutically acceptable excipients which are used may also be dissolved in the water-immiscible liquid.

The biphasic mixture may be held at elevated temperature for a period of time prior to the next step. For instance, the biphasic mixture may be incubated at around 37°C for up to 2 hours, for example for about 1 hour.

The biphasic mixture comprising the water-immiscible phase and aqueous phase is then subject to conditions which cause cross-linking of the polypeptides, typically by cross-linking cysteine residues in the polypeptide. The conditions used may also emulsify the mixture. Typically, for example, cross-linking is achieved by homogenising the bi-phasic mixture e.g. by applying high pressure and/or high shear stress conditions. Preferably, homogenisation is carried out by use of ultrasound. The high shear stress causes cross linking of the polypeptide, in particular by connecting cysteine groups within the polypeptide chains. It is understood that the homogenisation conditions may oxidise the sulfhydryl groups on a cysteine residue, or disrupt existing disulphide bonds, thereby allowing new disulphide bonds to form and generating the cross-linked shell structure. Water-immiscible phase remains physically trapped within the cross-linked polypeptide shell, providing the desired core-shell structure. The particles produced by the homogenisation step are generally substantially spherical in shape.

One example of a method for achieving homogenisation is to place an ultrasound horn at the interface of the aqueous and water-immiscible phases, and apply low-frequency ultrasound. Ultrasound may be applied at around 50% amplitude. For example, ultrasound may be applied for at least 1 minute, e.g. about 3 minutes. For example, particle formation has been achieved using a QSonica Q125 ultrasound probe (frequency: 20kHz; power: 125 watts) at 50% amplitude for 3 minutes.

An alternative method for achieving homogenisation is to use hydrodynamic cavitation, which uses high pressure to pump a liquid through a narrow orifice. The increase in flow velocity as the liquid passes through the orifice, and subsequent decrease in velocity once past the orifice, causes cavitation nuclei to grow and then collapse. Applying hydrodynamic cavitation to the biphasic mixture may cause cross-linking of cysteine residues and/or emulsification, leading to formation of particles. Hydrodynamic shearing is a further alternative method which may produce the same reactive oxygen species that reduce / oxidise cysteine residues that lead to particle formation. Other methods of sonication may also be used.

Spherical particles having a water-immiscible core and a polypeptide shell, which are produced by such homogenisation methods, are known in the art for use as drug delivery vehicles, and methods for their synthesis have been previously described (see Suslick K. S. and M. W. Grimstaff, Journal of the Americal Chemical Society, 1990, 112(21), p7807-7809, the contents of which are incorporated herein by reference). The particles of the invention may be made by such techniques or other methods of sonication, such as those described in US5439686, the contents of which are incorporated herein by reference.

The particles can be purified by suitable means and/or subject to size filtration. For example, particles can be separated from residual oil, solvent and protein by any suitable means, for example, by dialysis e.g. through a dialysis filter having a molecular weight cut off of appropriate size (e.g. 1M Da). Size filtration and/or centrifugation may also be used to limit the maximum size of the particles.

The particles may be suspended in a liquid medium for storage or use, e.g. water, or an aqueous solution, optionally a buffer solution.

### Compositions

The present invention provides a composition, typically a pharmaceutical composition, comprising one or more polypeptide particles as described herein. Compositions which contain a vaccine are referred to herein as vaccine compositions. Compositions which contain an adjuvant but do not contain a vaccine are referred to herein as adjuvant compositions. The composition may comprise a plurality of the polypeptide particles alone, or the particles may be provided together with one or more pharmaceutically acceptable carriers or diluents. Typically, compositions provided together with a carrier or diluent contain up to 85 wt% of a particle of the invention. More typically, such a composition contains up to 50 wt% of a particle of the invention. Preferred pharmaceutical or vaccine compositions are sterile and pyrogen free.

The compositions of the invention may comprise one or more different types of particle according to the invention. For instance, the composition may comprise first particles having a first type of polypeptide shell and second particles having a second (different) type of polypeptide shell. Thus, for instance, the compositions may comprise particles comprising two or more particles comprising two or more different antigen proteins relating to the same pathogen. Alternatively or additionally, the composition may comprise particles comprising pathogenic antigen proteins and separate particles comprising adjuvant. Alternatively or additionally, the composition may comprise particles comprising antigen proteins relating to different antigens, such that the composition may be used to vaccinate subjects against two or more different pathogens. Typically, the compositions of the invention comprise a single type of particle.

The compositions may comprise particles which themselves include an adjuvant, e.g. in the core of the particle, in the polypeptide shell, or as a ligand attached to the shell. Alternatively or additionally, further adjuvant(s) may be included in the composition. Suitable further adjuvants include an aluminum salt such as alum, aluminum hydroxide or aluminum phosphate, but may also be a salt of calcium (e.g. calcium phosphate hydroxide), iron or zinc, or may be an insoluble suspension of acylated tyrosine, or acylated sugars, or may be cationically or anionically derivatised saccharides, polyphosphazenes, biodegradable microspheres, monophosphoryl lipid A (MPL), lipopolysaccharides, lipid A derivatives (e.g. of reduced toxicity), 3-O-deacylated MPL [3D-MPL], detergents, e.g. quil A, Saponin, QS21, Freund's Complete Adjuvant, Freund's Incomplete Adjuvant (Difco Laboratories, Detroit, Mich.), Merck Adjuvant 65 (Merck and Company, Inc., Rahway, N.J.), AS-2 (Smith-Kline Beecham, Philadelphia, Pa.), CpG oligonucleotides, oils, e.g. squalene, mineral oils e.g. paraffin oil, food-based oils e.g. adjuvant 65 (derived from peanut oil), bacterial products, e.g. killed bacteria selected from *Bordatella pertussis, Mycobacterium bovis,* toxoids, bioadhesives and mucoadhesives, microparticles, liposomes, polyoxyethylene ether formulations, polyoxyethylene ester formulations, muramyl peptides or imidazoquinolone compounds (e.g. imiquamod and its homologues). Human immunomodulators suitable for use as adjuvants include cytokines such as interleukins (e.g. IL-1, IL-2, IL-4, IL-5, IL-6, IL-7, IL-12, etc), macrophage colony stimulating factor (M-CSF), tumour necrosis factor (TNF), granulocyte and macrophage colony stimulating factor (GM-CSF).

The particles in the composition may be substantially monodisperse. For instance, they may have a polydispersity index of 0.20 or less, e.g. 0.19 or less, 0.18 or less, 0.17 or less, 0.16 or less, or 0.15 or less. Greater monodispersity of the particles may provide a greater control over cavitation and thus improved acoustic tuning. Polydispersity may be determined by DLS.

The particles of the invention may be administered by any route conventionally used for vaccination. For instance, the particles may be administered by injection, by oral administration, intra-nasal administration or transdermal administration. The particles are typically administered by injection (subcutaneous, intradermal, intramuscular, intravenous, intratumoural, intrathecal etc.) or by oral or intra-nasal administration. In one aspect, the particles are administered by injection, preferably by intravenous, intramuscular, intradermal or subcutaneous injection.

In one embodiment, a composition provided together with a carrier or diluent is a liquid dispersion. Typically, water (preferably sterile water) is used as diluent for a liquid dispersion. Suspensions and emulsions may contain as carrier, for example a natural gum, agar, sodium alginate, pectin, methylcellulose, carboxymethylcellulose, or polyvinyl alcohol. The suspension or solutions for intramuscular injections may contain, together with the active compound, a pharmaceutically acceptable carrier, e.g. sterile water, olive oil, ethyl oleate, glycols, e.g. propylene glycol, and if desired, a suitable amount of lidocaine hydrochloride. Particles for administration in suspension form may be provided as lyophilised particles, for reconstitution in a suitable carrier (e.g. sterile water) prior to administration.

### Use of Particles

The particles and compositions of the invention may be used in a method of vaccination. The ability of the particles of the invention to elicit antibodies to a polypeptide incorporated into the polypeptide shell provides a particular benefit for use in a method of vaccination. Similarly, where the particles contain an adjuvant polypeptide, they may enhance the immune response to any co-administered vaccine. The particles may be administered together with a separate vaccine, e.g. an RNA or DNA vaccine.

Accordingly, the present invention provides a particle or composition as described herein for use in a method of vaccination. Also provided is a method of vaccination which comprises administering an effective amount of a particle or composition as described herein to a subject. Also provided is the use of a particle or composition as described herein in the manufacture of a medicament for use in a method of vaccination.

Particles which themselves generate an immunologic effect as a vaccine, may be provided together with an adjuvant and/or the particles themselves may additionally comprise an adjuvant. In one aspect, the particles comprise an adjuvant in the water-immiscible core of the particle. However, given the increased immunologic effect of the particles, an advantage of the use of the particles in a method of vaccination is that a sufficient immunologic effect may be achieved without administration of an adjuvant.

In alternative embodiments of the invention, the particles are administered together with (or the vaccine composition of the invention may comprise) a vaccine, e.g. a (ribo)nucleic acid vaccine. In some embodiments, the nucleic acid vaccine is an RNA or DNA vaccine. In this embodiment, the particles of the invention typically comprise an adjuvant.

Where an adjuvant is used in the method of vaccination, the adjuvant may be provided separately from the particles of the invention (i.e. in a separate composition), it may be provided in the same composition as the particles (but not as part of the particles themselves) or an adjuvant may be incorporated into the particles themselves. Two or more of these approaches may be combined.

Where an adjuvant is present in the particle itself, it may be comprised within the water-immiscible core, and/or an adjuvant polypeptide may be comprised within the polypeptide shell and/or an adjuvant may be provided as a ligand which is conjugated to the polypeptide shell of the particle of the invention.

In a particular aspect of the invention, the polypeptide particle or composition of the invention comprises an adjuvant (which may be present in the water-immiscible core, in the polypeptide shell, and/or as a ligand which is conjugated to the polypeptide shell) and the particle is administered together with, or the composition comprises, a vaccine, for example a (ribo)nucleic acid vaccine, in particular n RNA or DNA vaccine. For instance, the polypeptide particle or composition of the invention has a polypeptide shell which comprises an adjuvant polypeptide as described herein, and the particle or composition is administered together with a vaccine, for example a (ribo)nucleic acid vaccine, in particular an RNA or DNA vaccine.

The present invention therefore also provides a composition or kit comprising particles of the invention, together with a vaccine, e.g. a (ribo)nucleic acid vaccine, typically an RNA or DNA vaccine. The particles typically comprise an adjuvant, preferably the particles have a polypeptide shell which comprises an adjuvant polypeptide.

### Dosages

The dose of particles of the invention may be determined according to various parameters, especially according to the substance used; the age, weight and condition of the individual to be treated; and the required regimen. The amount of particle in each dose may, for example, be selected as an amount which induces an immune response. A physician will be able to determine the required dosage for any particular individual. The dose may be provided as a single dose or may be provided as multiple doses, for example taken at regular intervals, for example 2, 3 or 4 doses. Typically polypeptide-based treatments are administered in the range of 1 pg to 1 mg, more typically 1 pg to 10 µg for particle mediated delivery and 1 µg to I mg, more typically 1-100 µg, more typically 5-50 µg for other routes. Generally, it is expected that each dose will comprise 0.01-3 mg of polypeptide. An optimal amount for a particular treatment can be ascertained by studies involving observation of immune responses in subjects.

### Examples

### Particle Synthesis

Particles were prepared according to protocol A as follows:
3ml of a 50 mg/ml solution of protein is overlaid with 1ml of a water immiscible solution containing an oil, and optionally a volatile component and in some cases an additional agent, to produce a biphasic solution. The biphasic mixture is sealed in a glass vial and placed in an incubator at 37C for 1 hour.

An ultrasound horn is then placed at the interface of the two layers and low frequency ultrasound at 50% amplitude is applied for 3 minutes at 125 watts using QSonica Q125 at 50% amplitude. This produced an emulsion of substantially spherical particles comprising a core containing the water immiscible mixture and a protein shell.

Where volatile component was present in the core, the particles were further processed to remove the volatile component and thus produce ultrasound-responsive particles having surface indentations. The volatile solvent component of the particle core was thus removed under reduced pressure using a SpeedVac^{™} protein concentrator to provide a solution containing the particles.

The resulting indented particles are then separated from residual oil, solvent and protein by dialysis in a 1M Da molecular weight filter for 24 to 48 hours.

Particles were also produced according to a modified protocol B. Protocol B is identical to protocol A with the exception that the protein was at a lower concentration of 0.5mg protein in 666ul of water which was then overlaid with 333ul of the oil/volatile solvent layer.

The following particles were produced:

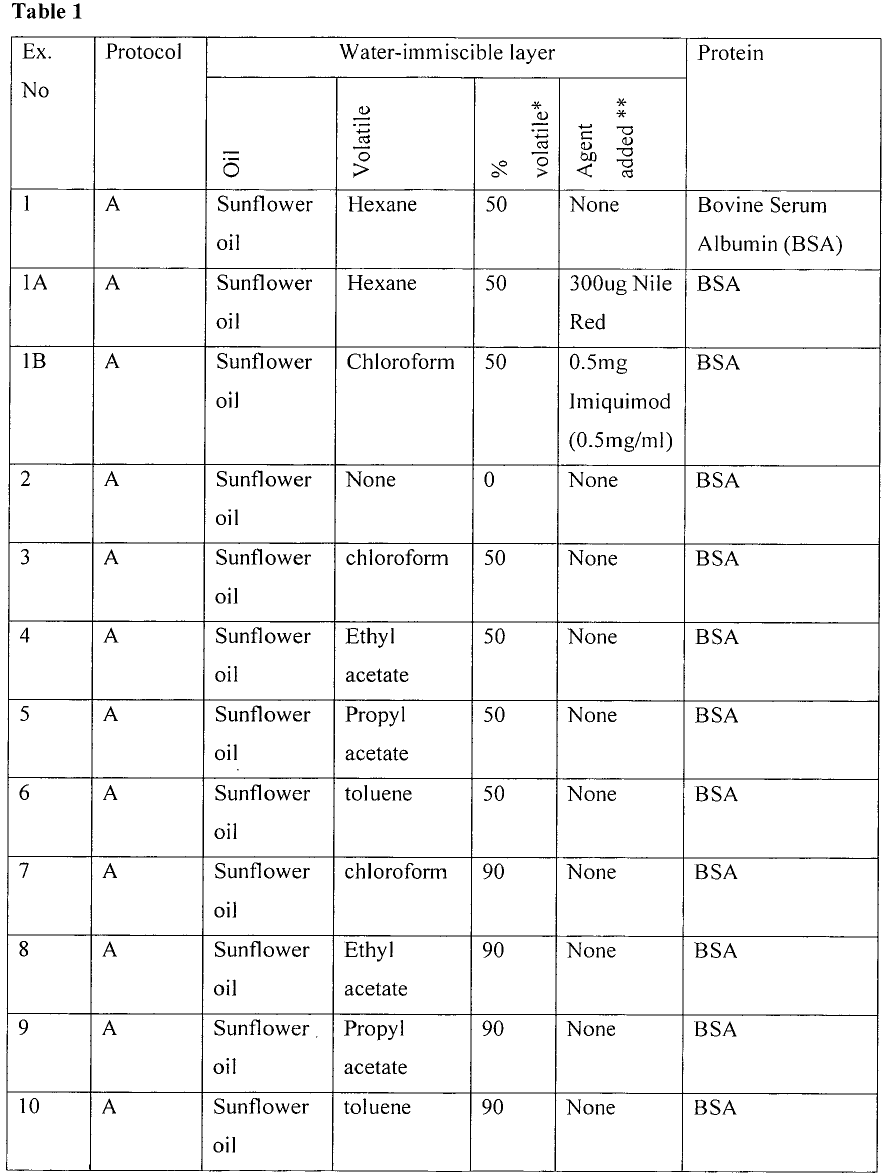

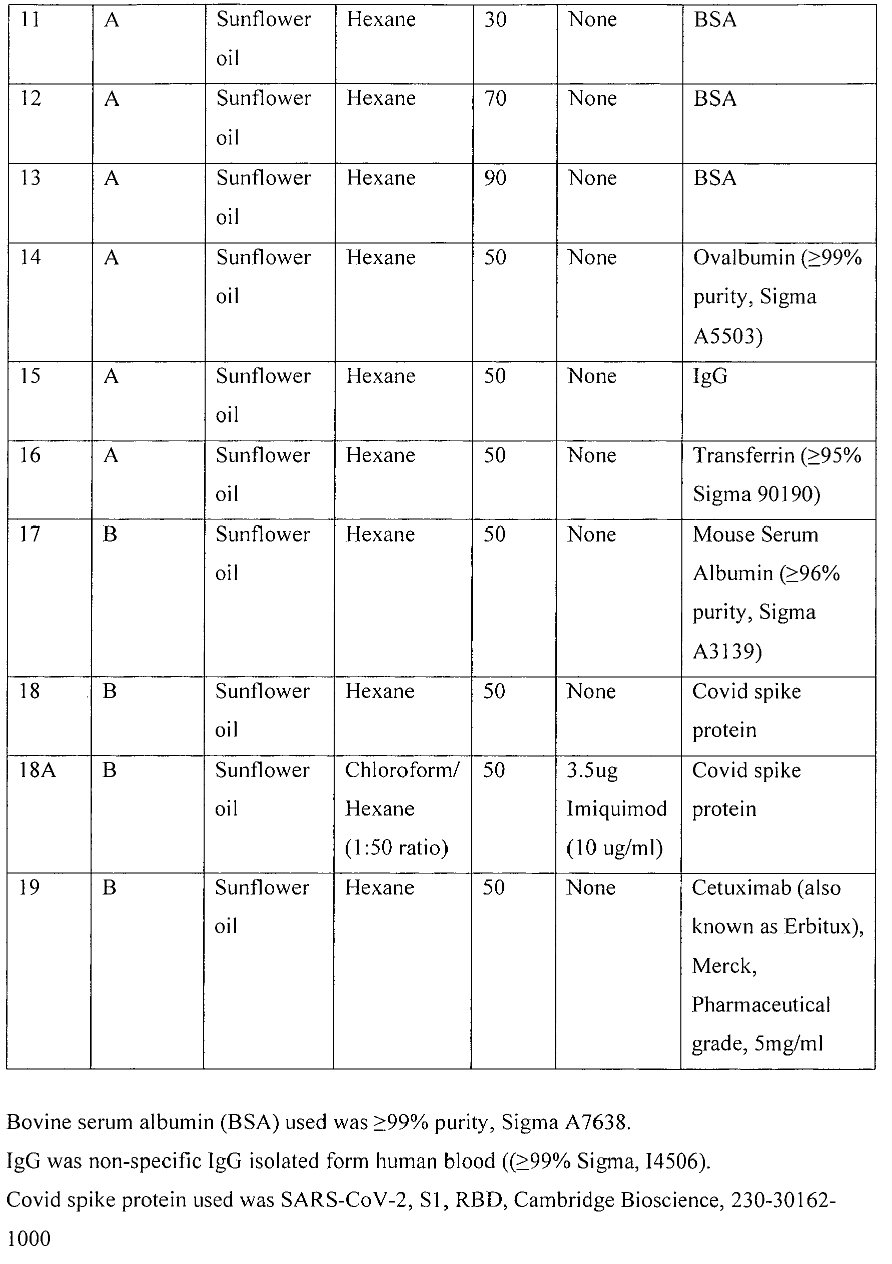

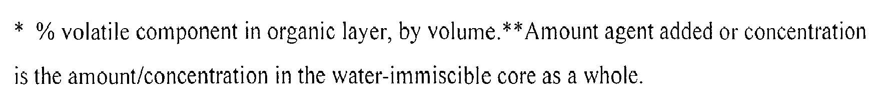

### Particle Analysis and Chemical Modification of Particle Surface

Particles were analysed by dynamic light scattering (DLS), scanning electron microscopy (SEM) and via spinning disc confocal microscopy following conjugation of the particle to fluorophores.

### SEM analysis

Instrumentation:
▪ Scanning electron microscopy (SEM) analysis was performed using a Zeiss Sigma 300 Field Emission Gun Scanning Electron Microscope (FEG-SEM).

Method:
▪ Typically a 1 in 1000 dilution of a stock solution was made. This is fixed with 1% glutaraldehyde for 2h, and 10ul added to a black carbon tape and coated with gold before imaging

### Spinning disc confocal microscopy

Instrumentation:
▪ An Andor Dragonfly spinning disc confocal microscope was used to generate high resolution Z stacks that were then compiled into 3D reconstructions of protein particles

Method:
▪ Particles were reacted with CY-5^{®} N-hydroxysuccinimide (NHS) or AlexaFluor^{™} 488 N-hydroxysuccinimide (NHS) for 1h. Dialysis for 16h using a 10kDa molecular weight cassette was used to remove residual unconjugated dye prior to imaging.

### Dynamic Light Scattering

Instrumentation:
▪ Size distributions were measured using DLS (ZetaSizerNano, Malvern) with a red laser. Each sample was tested with three measurements of approximately 10 runs.

Method:
▪ 10ul of a particle sample prepared as set out above was diluted to 1ml with Millipore water and analysed in a standard 1ml cuvette.

Figures 2a to 2b and Figure 2c show particles of Example 1 above via 3D confocal microscopy and SEM respectively. Ligands successfully conjugated to particles via NHS-lysine conjugation, and provided visibility of particles via confocal microscopy as shown in Figure 2a.

This experiment confirms that upon forming particles, the protein retains the ability to undergo standard protein conjugation reactions, in this case reaction with chemistry targeting lysine residues. The particles became fluorescent following conjugation of two different lysine reacting dyes.

Figure 2d shows an SEM image of substantially spherical particles produced according to Example 2.

### Addition of Compound to Oil Core

Example 1A demonstrates that compounds dissolved in the oil layer during synthesis are incorporated into the oil core of the particles.

Protocol A was followed to produce the particles as set out in Example 1 above, but 300ug of Nile Red oil soluble fluorophore was incorporated into the oil layer prior to application of ultrasound to form the particles. Any residual fluorophore was removed from the particles by dialysis.

Fluorescent confocal microscopy was used to image the particles. This confirmed the presence of Nile Red inside the core of the particles.

Example 1B describes the production of particles containing the adjuvant imiquimod. imiquimod was included in the chloroform volatile component at a concentration of 1 mg/ml and mixed with the sunflower oil prior to particle production. Protein particles were produced according to the standard protocol A.

Purified protein particles were digested with beta mercaptoethanol and proteinase K for 1 hour at 60C to break up the external protein shell and release the inner core. The core content was then analysed by UPLC to confirm the presence of imiquimod. UPLC analysis was carried out substantially as described in the method by Balireddi et al (Hournal of Chrom Science, Volume 57, Issue 3, March 2019, p249-257).

UPLC analysis confirmed the presence of imiquimod as shown in Figure 2f. Fig 2f(I) shows the imiquimod standard curve and the amounts of imiquimod detected in the particle core. Amounts in excess of 1 ug/ml were detected. Fig 2f(II) shows the UV absorbance profile, which corresponds to the characteristic absorbance profile of imiquimod.

### Variation of Polypeptide, optionally with adjuvant in oil core

Variation of the protein to incorporate a variety of different proteins into the particle was achieved as set out in Examples 14 to 19. In particular, Example 14 demonstrates the production of a particle having an adjuvant protein, ovalbumin, in the polypeptide shell, whilst Example 18 demonstrates the manufacture of particles having a pathogenic antigen protein, covid spike protein, in the polypeptide shell.

Example 18A demonstrates the production of particles having covid spike protein as the polypeptide shell, and also having adjuvant in the water-immiscible core. In this example, protocol B was followed, and modified as follows:
Imiquimod was dissolved in chloroform at 0.5 mg/ml. 7ul (1 in 50 dilution) was then added to 326ul of the hexane/oil layer to provide a total volume of 333ul of oil layer. This oil layer was then overlaid on a solution of Covid spike protein to provide the biphasic mixture. Particles were produced in accordance with protocol B. The maximum content of imiquimod was therefore 1ug/100ul in the resulting particle suspension.

### Ability of Particles to retain functionality of protein from which they are derived

The aim of this study was to assess the ability of particles to retain functional binding to a receptor following particle formation.

Particles of example 19 containing cetuximab in the polypeptide shell (prepared according to protocol A as set out above) were dialysed for 48h using a 1M Da molecular weight filter to remove any free (non-particle bound) Cetuximab. An ELISA specific to the protein EGFR (a validated binding target for Cetuximab) was undertaken to compare the binding efficiency of equal amounts (mg/mg) protein of native Cetuximab to Cetuximab particles.

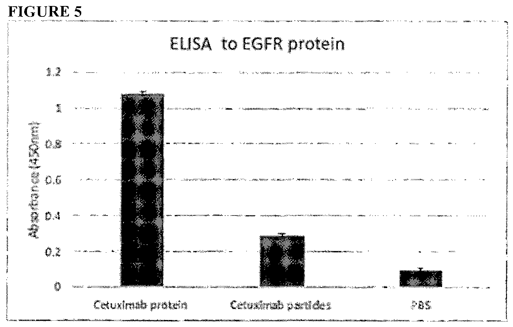

The results are depicted in Figure 5. Figure 5 demonstrates that particles composed of Cetuximab retain an ability to bind to EGFR. This supports the understanding that antigen proteins or adjuvant proteins forming part of the polypeptide shell are able to retain their therapeutic efficacy after particle formation. The reduced level of binding compared to native Cetuximab in this experiment is understood to relate to steric hindrance of the particles, and a lack of optimisation.

### In Vivo Cavitation Ultrasound Methodology

In vivo transdermal particle cavitation in the experiments described below was carried out by using a source transducer (117D-01, Sonic Concepts, USA) to initiate cavitation. The transducer was fitted with a Perspex coupling cone (used to efficiently transmit ultrasound from the source to the therapy target), filled with degassed water and covered with an acoustically transparent Mylar plastic. The coupling cone shape was designed to align with the outer envelope of the focused ultrasound beam. A formulation holder, containing particle solution, was placed on the murine skin and the source transducer was placed on top of this, with the Mylar plastic acoustic window positioned to prevent air entering the acoustic path. A single element spherically focused ultrasound transducer acting as passive acoustic detector (PCD) was coaxially located inside the source transducer, to allow the cavitation signal to be recorded. The PCD signal was passed through a 2.0 MHz high pass filter and amplified with a gain of 25 by a pre-amplifier (SR445A, Stanford Research Systems, USA). The signal was then digitised and monitored in real time by an 12-bit digital oscilloscope (Handyscope HS3 100 MHz, TiePie Engineering, The Netherlands) and the data was saved on an external hard drive (Portable SSD T5, Samsung, Korea) (Figure 2). This specific cavitation detection setup has been documented in Gray et al, IEEE Trans. UFFC 65(2) 2018, pp 258-267. The set-up is schematically depicted in Figure 3.

### Particle Administration and Assessment of Immune Response

Particles according to the invention were applied to mice. Immune response to the protein delivered was assessed.

8 mice were used in the experiment, and each mouse received two separate particle preparations, one applied to each flank. Thus, 16 experiments were carried out in total as follows:
Group 1: 4 x Protein particles + US
Group 2: 3 x Protein particles without US
Group 3: 3 x protein subcutaneous injection
Group 4: 3 x protein Intradermal injection
Group 5: 3 x protein Intramuscular injection
Protein particles used were particles produced in accordance with Example 1 above. The particles were administered to each mouse either by direct application to the mouse flank (with or without application of ultrasound), or by injection as indicated above. The total protein content of each component administered was 9mg. It is important to note that for injection, the full 9mg is delivered to the mouse, whereas for transdermal administration, only a fraction of the 9mg protein is delivered. Where ultrasound was used, this was carried out as discussed in *"In Vivo Cavitation Ultrasound Methodology"* above. Exposure time was 10 minutes.

Following administration of the particles in the designated manner for each group, immune response to BSA protein was determined by carrying out ELISA analysis to study immune response to BSA following delivery.

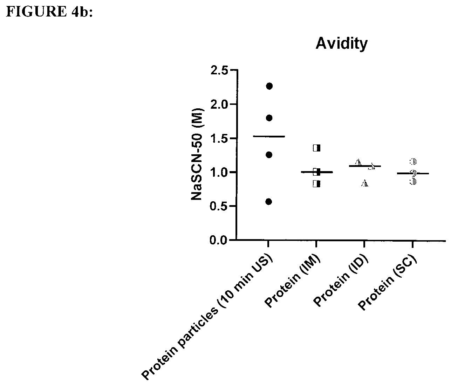

Results are depicted in Figure 4a. Immune response to BSA in the transdermal group (Group 1) was comparable to that of the injection groups, showing successful delivery of BSA via the transdermal route and successful generation of immune response to the administered protein. This level of immune response is surprising, given the lower amount of particle administered to each mouse. Figure 4(b) also shows that the transdermal group generated particles with the highest avidity.

### Use as Vaccine

The aim of this experiment was to establish that inventive particles comprising an antigen protein (Covid19 spike protein) and containing the adjuvent imiquimod inside the oil core could enhance the immune response and compare with that of non imiquimod particles, native protein or native protein co-injected with imiquimod.

Mice were treated by administration of a composition as set out below. Each mouse received two separate particle preparations, one applied to each flank. The preparations applied were as follows. All preparations were made up in PBS:
- PBS intradermal (ID) injection
- Covid19 protein (5ug) ID injection
- Covid19 protein (5ug) + imiquimod (0.1ug) ID injection
- Covid19 protein particles (Ex 18, indented particles) (providing 5ug protein) ID injection
- Covid19 protein particles with imiquimod core (Ex 18A, indented particles) (providing 5ug protein and 0.1ug imiquimod) ID injection
- Covid19 protein particles with imiquimod core (Ex 18A, indented particles) (providing 5ug protein and 0.1ug imiquimod) transdermal + 10 minute US exposure

Covid spike protein used was SARS-CoV-2, S1, RBD, Cambridge Bioscience, 230-30162-1000. Protein was either used in native form, or prepared into particles produced in accordance with Example 18 or 18A above. Particles were administered suspended in PBS and in an identical form for both intradermal injection and transdermal administration.

The compositions set out above were administered to each mouse either by direct application to the mouse flank, or by intradermal injection as indicated above. The total protein content of each component administered was 5ug. It is important to note that for injection, the full 5ug is delivered to the mouse, whereas for transdermal administration, only a fraction of the 5ug protein is delivered. Total imiquimod content of samples, where relevant, was 0.1ug. The same amount of imiquimod is present whether the imiquimod is included in the core of the particle or provided separately. Where ultrasound was used, this was carried out as discussed in *"In Vivo Cavitation Ultrasound Methodology"* above.

Mouse serum was sampled at intervals after administration of protein. An ELISA assay specific to covid spike protein RBD domain was used to assess generation of antibodies. Figure 6 shows the results of a day-21 ELISA assay. Protein particles with an imiquimod core generated higher levels of antibodies than native spike proteins delivered intradermally. Given the lower amount of protein which is expected to be delivered via this route, the greater level of immune response is surprising.

## Claims

1. A vaccine composition comprising a plurality of polypeptide particles and one or more pharmaceutically acceptable carriers or diluents, wherein the particles comprise:
- a water immiscible liquid core optionally comprising one or more adjuvants; and
- a polypeptide shell.

2. A vaccine composition according to claim 1, wherein the polypeptide comprises a pathogenic antigen protein and/or wherein the composition further comprises a vaccine, preferably a DNA or RNA vaccine.

3. A vaccine composition according to claim 1 or claim 2, wherein any indentations on the surface of the polypeptide particles have a maximum dimension of 50nm, preferably wherein the particles are substantially spherical.

4. A vaccine composition according to any one of the preceding claims, wherein the polypeptide particles have a mean particle size in the range of from 100nm to 1000nm, preferably from 200nm to 700nm.

5. A vaccine composition according to any one of the preceding claims, wherein the water-immiscible core comprises one or more adjuvants, preferably wherein the one or more adjuvants are selected from imiquimod and squalene.

6. A vaccine composition according to any one of the preceding claims, wherein the polypeptide comprises a pathogenic antigen protein, preferably wherein the pathogenic antigen protein is selected from a covid spike protein, HepB surface antigen protein, hemagglutinin, an influenza neuraminidase, filamentous hemagglutinin, pneumococcal surface protein A, Neisserial adhesin A, Neisserial Heparin Binding Antigen, factor H binding protein, and HPV-16 E6 or E7 fusion protein.

7. A vaccine composition according to any one of the preceding claims, wherein the polypeptide comprises an adjuvant polypeptide, preferably wherein the adjuvant polypeptide is one or more polypeptides selected from non-human albumin proteins, sFLT ligand, cytokines and chemokines, macrophage colony stimulating factor (M-CSF), tumour necrosis factor (TNF), and granulocyte and macrophage colony stimulating factor (GM-CSF)

8. A polypeptide particle comprising:
- a water immiscible liquid core optionally comprising one or more adjuvants, the adjuvants being as defined in claim 5; and
- a polypeptide shell comprising a pathogenic antigen protein as defined in claim 6;
preferably wherein any indentations on the surface of the polypeptide particle have a maximum dimension of 50nm.

9. A polypeptide particle comprising:
- a water immiscible liquid core optionally comprising one or more adjuvants, the adjuvants being as defined in claim 5; and
- a polypeptide shell comprising an adjuvant polypeptide as defined in claim 7;
preferably wherein any indentations on the surface of the polypeptide particle have a maximum dimension of 50nm.

10. A pharmaceutical adjuvant composition comprising a plurality of particles according to claim 9, together with one or more pharmaceutically acceptable carriers or diluents.

11. A polypeptide particle or composition according to any one of claims 8 to 10, which particle is as defined in any one of claims 3 or 4.

12. A method of producing a polypeptide particle according to any one of claims 8, 9 or 11, comprising:
- providing a water-immiscible phase optionally comprising one or more adjuvants as defined in claim 5;
- mixing said water-immiscible phase with an aqueous solution of at least one polypeptide, wherein the at least one polypeptide comprises a pathogenic antigen protein as defined in claim 6 and/or an adjuvant polypeptide as defined in claim 7;
- cross-linking the polypeptide to generate a particle having a core comprising the water-immiscible phase and a shell comprising the at least one polypeptide; and optionally
- purifying the particles.

13. A polypeptide particle or composition according to any one of claims 1 to 11, for use in a method of vaccination.

14. A polypeptide particle or composition for use according to claim 18, wherein the polypeptide particle or composition is administered by injection, by oral administration or by intra-nasal administration, preferably wherein the polypeptide particle or composition is administered by intravenous, intradermal, intramuscular or subcutaneous injection.

15. A polypeptide particle or composition for use according to claim 13 or claim 14, wherein the polypeptide particle or composition is administered together with a DNA or RNA vaccine.
